# EUROPEAN PATENT SPECIFICATION

(11) **EP 1 320 343 B1**
(45) Date of publication and mention of the grant of the patent: **09.02.2005**
(21) Application number: 01972259.4
(22) Date of filing: 28.09.2001
(51) Int. Cl.: A61F 13/02

(54) **ADAPTABLE DRESSINGS**
ANPASSBARES WUNDPFLASTER
PANSEMENTS ADAPTABLES

(30) Priority: 29.09.2000 GB 0023925
(43) Date of publication of application: 25.06.2003
(73) Proprietor: Johnson & Johnson Medical Ltd., Edinburgh EH2 4NH (GB)
(72) Inventor: STOW, Martin, William, Skipton, North Yorkshire BD23 1UZ (GB); DALE, Cynthia, Mary, Skipton, North Yorkshire BD23 2DX (GB)
(74) Representative: James, Anthony Christopher W.P.
(86) International application number: PCT/GB2001/004362
(87) International publication number: WO 2002/026180

(56) References cited:
- DE-B- 1 023 190
- GB-A- 272 423
- US-A- 4 631 227

## Description

The invention relates to adhesive dressings, especially wound dressings, and in particular to sacral dressings for the treatment and prevention of sores and ulcers of the sacrum and other difficult to treat areas of the body.

Adhesive dressings are known for the treatment of wounds, including ulcers and pressure sores. Adhesive dressings are also applied to intact skin to protect the skin, and for the percutaneous administration of active agents. Typical adhesive dressings are the so-called island dressings, in which a smaller area of absorbent wound dressing material (the island) is supported on an adhesive-coated, preferably microorganism-resistant backing sheet that provides an adhesive-coated margin around the island.

DE-A-1023190 describes adhesive dressings especially for attachment to a finger. The dressings comprise an adhesive-coated backing sheet in the form of a strip, and an absorbent island offset lengthways from the center of the strip.

The invention provides an adhesive dressing comprising: a backing sheet; a layer of pressure-sensitive adhesive coated on the backing sheet; and an absorbent island adhered to the backing sheet on the same side as the adhesive layer, wherein the island has a smaller area than the backing sheet such that an adhesive-coated margin of the backing sheet extends around the island, wherein the island is offset from the center of the backing sheet, characterised in that a potential region to be removed is indicated in a wide part of the adhesive-coated margin.

The backing sheet is substantially liquid-impermeable. That is to say, it is substantially continuous and free from macroscopic apertures or slits that could allow the passage of liquid water or wound fluid. The backing sheet is preferably semipermeable. That is to say, the backing sheet is preferably permeable to water vapour, but not permeable to liquid water or wound exudate. Preferably, the backing sheet is also microorganism-impermeable. Suitable continuous conformable backing sheets will preferably have a moisture vapor transmission rate (MVTR) of the backing sheet alone of 300 to 5000 g/m²/24hrs, preferably 500 to 2000 g/m²/24hrs at 37.5 °C at 100% to 10% relative humidity difference. The backing sheet thickness is preferably in the range of 10 to 1000 micrometers, more preferably 100 to 500 micrometers.

Suitable polymers for forming the backing sheet include polyurethanes and poly alkoxyalkyl acrylates and methacrylates such as those disclosed in GB-A-1280631. Preferably, the backing sheet comprises a continuous layer of a high density blocked polyurethane foam that is predominantly closed-cell. A suitable backing sheet material is the polyurethane film available under the Registered Trade Mark ESTANE 5714F. Preferably, the backing sheet is substantially inelastic. Preferably, the sheet exhibits an elastic recovery at 50% strain of less than 90%, preferably less than 75%, more preferably less than 50%.

The backing sheet may have any shape, such as circular, oval or rectangular. Preferably, the backing sheet is shaped with at least one line of symmetry, and preferably the island is offset along a line of symmetry of the backing sheet. More preferably, the backing sheet is substantially rectangular (including square). Preferably, the backing sheet is substantially rectangular and has dimensions of from 5 to 25 cm by from 5 to 25 cm, more preferably from 10 to 20 cm by from 10 to 20 cm.

The adhesive layer should be moisture vapor transmitting and/or patterned to allow passage of water vapor therethrough. The adhesive layer is preferably a continuous moisture vapor transmitting, pressure-sensitive adhesive layer of the type conventionally used for island-type wound dressings, for example, a pressure sensitive adhesive based on acrylate ester copolymers, polyvinyl ethyl ether and polyurethane as described for example in GB-A-1280631. The basis weight of the adhesive layer is preferably 20 to 250 g/m², and more preferably 50 to 150 g/m². Polyurethane-based pressure sensitive adhesives are preferred.

The adhesive layer extends outwardly from the absorbent island to form an adhesive-coated margin on the backing sheet around the adhesive island as in a conventional island dressing. Preferably, the wound facing surface of the absorbent island itself is substantially free of the adhesive present on the backing sheet, and more preferably it is substantially free of any adhesive.

The absorbent island may comprise any of the layers conventionally used for absorbing wound fluids, serum or blood in the wound healing art, including gauzes, nonwoven fabrics, superabsorbents, hydrogels and mixtures thereof. Preferably, the absorbent island comprises a layer of absorbent foam, such as an open celled hydrophilic polyurethane foam prepared in accordance with EP-A-0541391, the entire content of which is expressly incorporated herein by reference. In other embodiments, the absorbent island may comprise a nonwoven fibrous web, for example a carded web of viscose staple fibers. The basis weight of the absorbent island may be in the range of 50-500g/m², such as 100-400g/m². The absorbent island may comprise more than one layer. For example, it may comprise an absorbent layer laminated to a non-adherent, liquid-permeable wound contacting top sheet. The uncompressed thickness of the absorbent island may be in the range of from 0.5mm to 10mm, such as 1mm to 4mm. Preferably, the uncompressed thickness of the island is less than 3mm. The free (uncompressed) liquid absorbency of the island measured for physiological saline may be in the range of 5 to 30 g/g at 25°.

Preferably, the absorbent island has at least one dimension greater than 4cm, preferably 5 to 10cm. The shape of the island likewise may be any shape, such as circular, oval or rectangular, and may be the same or different from the shape of the backing sheet. The area of the absorbent island is typically in the range of from 1cm² to 200cm², more preferably from 4cm² to 100cm². In certain embodiments, such as sacral dressings, the area of the absorbent layer is preferably greater than 16cm², more preferably greater than 20cm².

Conventional island-type dressings have the island centered on the backing sheet. That is to say, the center of gravity of the island is at, or very close to, the center of gravity of the backing sheet. In contrast, the dressings according to the present invention have the center of gravity of the island offset from the center of gravity of the backing sheet. The offset island of the present invention provides the dressing with at least one wider-than-average region of the adhesive-coated margin. This region can be shaped, e.g. by cutting or by punching or tearing out part of the region, to profile it so that the dressing conforms accurately to a region of the body to which the dressing is to be applied.

Preferably, the center of gravity of the island is offset by from 1 to 8 cm., more preferably from 2 to 6 cm, from the center of gravity of the backing sheet. Preferably, the mean width of the adhesive-coated margin is from 1 to 5 cm.

The dressings according to the present invention are especially useful for difficult-to-dress areas of the body such as the sacrum, elbows, knees or heels. Preferably, the dressing is a sacral wound dressing. Such dressings are generally relatively large-area dressings of 100-600 cm², preferably 150-400 cm², and must be shaped to conform to the differing regions of different patients. Preferably, the sacral dressing is substantially rectangular (including square) as hereinbefore described.

As already noted, it is a feature of the dressings according to the invention that the wide margin region can be cut out to adapt the shape of the dressing to a body region. Therefore, at least one edge of the backing sheet in the dressings according to the present invention may be non-linear as a result of such cutting out. Furthermore, a potential cut-out region may be indicated in a wide part of the adhesive-coated margin. For example, one or more alternative cut-out lines may be marked on the backing sheet (or on a cover sheet as described below) by ink or embossing. Alternatively or addtionally the cut-out lines may be provided with perforations or may be die-cut to enable the regions to be torn or pulled out by a user. In certain embodiments the cut-out lines define a notch, for example a V- or U-shaped notch in the wide margin of the backing sheet.

it remains desirable that a minimum adhesive-coated margin should remain around the island after the cutting out in order to ensure secure adhesion of the dressing. Therefore, preferably, a minimum adhesive margin width is indicated in the wide part of the adhesive-coated margin, for example by inking or embossing. Preferably, the minimum margin width is from 1 to 6 cm, preferably from 1.5 to 4 cm.

Preferably, the adhesive dressings according to the invention further comprise at least one release-coated cover sheet covering the island and the adhesive-coated margin. More preferably, the dressings comprise two edge cover sheets covering opposed edges of the dressing and a central cover sheet extending between the edge cover sheets, preferably substantially as described in EP-A-0117632.

Preferably, the adhesive dressing according to the present invention is suitable for medical use. Preferably, the dressing is sterile and preferably it is packaged in a microorganism-impermeable container.

The present invention also provides a method of production of a wound dressing according to claim 14 comprising the steps of: providing a wound dressing according to any aspect of the present invention, followed by removing the indicated region from the wide part of the adhesive-coated margin to improve the conformity of the dressing to a region of a patient.

Preferably, the dressing is a substantially rectangular sacral dressing, the island is displaced along a short axis of the rectangle, and a notch is removed of the wide adhesive coated margin in the region of the short axis.

An embodiment of the present invention will now be described in more detail, with reference to the accompanying drawings, in which:
Figure 1 shows a top plan view of a dressing according to the present invention with the underlying island shown in phantom; and
Figure 2 shows a bottom perspective view of the dressing of Figure 1, with the central cover sheet portion lifted to show underlying detail.

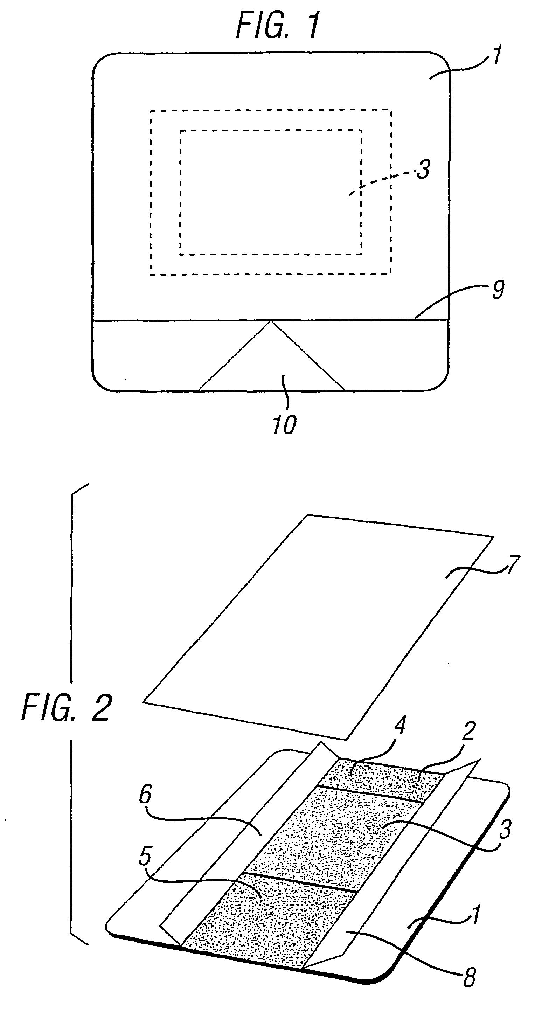

Referring to Figure 1, the backing sheet 1 is formed from a microporous polyurethane sponge film of the kind conventionally used to provide a breathable but microorganism-impermeable backing to island wound dressings. The backing sheet 1 is rectangular with rounded corners. The dimensions are approximately 150 mm by 150 mm, which makes the dressing suitable for protection of a human sacral region.

Referring to Figure 2, a lower surface of the backing sheet 1 is coated with a layer 2 of a medical grade pressure-sensitive adhesive. An absorbent island 3 is formed from a layer of nonwoven textile absorbent and a porous top sheet covering the absorbent layer and extending slightly beyond the edges of the absorbent layer. The porous top layer may itself be a polyurethane foam or other absorbent primry wound contact layer. Both layers of the island 3 are adhered to the backing sheet by the pressure sensitive adhesive. The adhesive-coated margin around the island has an average width of about 20-50 mm, but the island 3 is offset along the short edge of the backing sheet rectangle so that on this axis there is a normal margin 4 of about 30 mm width, and a wide margin 5 of about 50 mm width opposite the normal margin 4.

The island and the adhesive-coated margin are covered by three release-coated cover sheets 6,7,8 arranged as shown and as described in EP-A-0117632.

A line 9 is embossed or inked on the backing sheet 1 to indicate the region of the backing sheet that has a wide margin, and to show the limit beyond which cutting the backing sheet would intrude into a minimum margin width around the island. A series of notches 10 of different sizes are also marked on the backing sheet to indicate where the backing sheet could be cut away to provide conformity with a sacral region of a patient. Alternatively or additionally, these markings may be provided on the release sheets 6,7,8.

In use, a care giver cuts the wide margin 5 of the dressing using scissors or a knife to shape the dressing for an optimum sacral fit to a given patient. Alternatively, the care giver may tear or punch out a predefined region of the dressing. The care giver then removes the central release paper 7 and, while grasping the dressing by the side edges having the edge release papers 6,8 still applied thereto, applies the dressing to the sacrum of the patient. The care giver then removes the edge release papers 6,8 and smooths the edges of the dressing down onto the patient's skin.

The dressings and method according to the present invention have improved conformability to difficult to treat regions of the body, such as the sacrum. This greatly improves the wear time of the dressings. It also improves the comfort of the dressing, reduces leakage and allows the dressing to be customized to different patients.

The above embodiment has been described by way of example only. Many other examples of the present invention as defined in the accompanying claims will be apparent to the skilled reader.

## Claims

1. An adhesive dressing comprising: a backing sheet (1); a layer of pressure-sensitive adhesive (2) coated on the backing sheet; and an absorbent island (3) adhered to the backing sheet on the same side as the adhesive layer, wherein the island has a smaller area than the backing sheet (1) such that an adhesive-coated margin (4, 5) of the backing sheet extends around the island, wherein the island (3) is offset from the center of the backing sheet, **characterised in that** a potential region to be removed (10) is indicated in a wide part (5) of the adhesive-coated margin.

2. An adhesive dressing according to claim 1, wherein the island (3) is offset by from 1 to 8 cm.

3. An adhesive dressing according to claim 1 or 2, wherein the mean width of the adhesive-coated margin (4, 5) is from 1 to 6 cm.

4. An adhesive dressing according to any preceding claim, wherein the backing sheet (1) is substantially rectangular.

5. An adhesive dressing according to claim 4, wherein the backing sheet (1) has dimensions of from 5 to 25 cm by from 5 to 25 cm.

6. An adhesive dressing according to claim 5, wherein the backing sheet (1) has dimensions of from 10 to 20 cm by from 10 to 20 cm.

7. An adhesive dressing according to any preceding claim, wherein the dressing is a sacral wound dressing.

8. An adhesive dressing according to any preceding claim, wherein at least one edge of the backing sheet (1) is non-linear.

9. An adhesive dressing according to any preceding claim, wherein the backing sheet (1) is shaped with at least one line of symmetry, and the island (3) is offset along a line of symmetry of the backing sheet.

10. An adhesive dressing according to any preceding claim, wherein a minimum adhesive margin width is indicated (9) in the wide part (5) of the adhesive-coated margin (4, 5).

11. An adhesive dressing according to any preceding claim, further comprising at least one release-coated cover sheet covering the island (3) and the adhesive-coated margin (4, 5).

12. An adhesive dressing according to claim 11, comprising two edge cover sheets (6, 8) covering opposed edges of the dressing and a central cover sheet (7) extending between the edge cover sheets.

13. An adhesive dressing according to any preceding claim, wherein the dressing is sterile and packaged.

14. A method of production of a wound dressing comprising the steps of:
providing an adhesive dressing comprising: a backing sheet (1); a layer of pressure-sensitive (2) adhesive coated on the backing sheet; and an absorbent island (3) adhered to the backing sheet on the same side as the adhesive layer, wherein the island has a smaller area than the backing sheet such that an adhesive-coated margin (4, 5) of the backing sheet extends around the island, wherein the island is offset from the center of the backing sheet to provide a wide region (5) of the adhesive-coated margin; **characterised in that** a potential region to be removed (10) is indicated in the wide region (5) of the adhesive-coated margin, and said method further comprises removing said region (10) to improve the conformity of the dressing to a region of a patient.

15. A method according to claim 14, wherein the dressing is a substantially rectangular sacral dressing, the island (3) is displaced along a short axis of the rectangle, and said region (10) is a notch in the wide adhesive coated margin in the region of the short axis.

## Patentansprüche

1. Pflasterverband, der Folgendes umfaßt: eine dünne Trägerschicht (1), eine auf die Trägerschicht aufgetragene Lage aus Haftklebemittel (2) und eine absorbierende Insel (3), die auf der gleichen Seite wie die Klebemittellage an der Trägerschicht angehaftet ist, wobei die Insel einen kleineren Bereich aufweist als die Trägerschicht (1), so daß ein mit Klebemittel beschichteter Rand (4, 5) der Trägerschicht um die Insel (3) herum verläuft, wobei die Insel von dem Zentrum der Trägerschicht versetzt ist, **dadurch gekennzeichnet, daß**
in einem breiten Teil (5) des mit Klebemittel beschichteten Randes ein potenziell zu entfernendes Gebiet (10) kenntlich gemacht ist.

2. Pflasterverband nach Anspruch 1, **dadurch gekennzeichnet, daß**
die Insel (3) um 1 bis 8 cm versetzt ist.

3. Pflasterverband nach Anspruch 1 oder 2, **dadurch gekennzeichnet, daß**
die mittlere Breite des mit Klebemittel beschichteten Randes (4, 5) 1 bis 6 cm beträgt.

4. Pflasterverband nach einem der vorhergehenden Ansprüche, **dadurch gekennzeichnet, daß**
die Trägerschicht (1) im Wesentlichen rechteckig ist.

5. Pflasterverband nach Anspruch 4, **dadurch gekennzeichnet, daß**
die Trägerschicht (1) Abmessungen von 5 bis 25 cm mal 5 bis 25 cm aufweist.

6. Pflasterverband nach Anspruch 5, **dadurch gekennzeichnet, daß**
die Trägerschicht (1) Abmessungen von 10 bis 20 cm mal 10 bis 20 cm aufweist.

7. Pflasterverband nach einem der vorhergehenden Ansprüche, **dadurch gekennzeichnet, daß**
es sich um einen Sakralwundverband handelt.

8. Pflasterverband nach einem der vorhergehenden Ansprüche, **dadurch gekennzeichnet, daß**
mindestens eine Kante der Trägerschicht (1) nicht linear ist.

9. Pflasterverband nach einem der vorhergehenden Ansprüche, **dadurch gekennzeichnet, daß**
die Trägerschicht (1) mit mindestens einer Symmetrielinie geformt und die Insel (3) an einer Symmetrielinie der Trägerschicht entlang versetzt ist.

10. Pflasterverband nach einem der vorhergehenden Ansprüche, **dadurch gekennzeichnet, daß**
in dem breiten Teil (5) des mit Klebemittel beschichteten Randes (4, 5) eine minimale Breite des Klebemittelrandes kenntlich gemacht (9) ist.

11. Pflasterverband nach einem der vorhergehenden Ansprüche, **dadurch gekennzeichnet, daß**
der Pflasterverband ferner mindestens eine mit Trennmittel beschichtete Abdeckschicht umfaßt, die die Insel (3) und den mit Klebemittel beschichteten Rand (4, 5) abdeckt.

12. Pflasterverband nach Anspruch 11, **dadurch gekennzeichnet, daß**
der Pflasterverband zwei Kantenabdeckschichten (6, 8) umfaßt, die sich gegenüberliegende Kanten des Verbandes abdecken, und eine zentrale Abdeckschicht (7), die sich zwischen den Kantenabdeckschichten erstreckt.

13. Pflasterverband nach einem der vorhergehenden Ansprüche, **dadurch gekennzeichnet, daß**
der Verband steril und verpackt ist.

14. Verfahren zur Herstellung eines Wundverbandes, das folgende Schritte umfaßt:
Bereitstellen eines Pflasterverbandes, der Folgendes umfaßt: eine Trägerschicht (1), eine auf die Trägerschicht aufgetragene Lage aus Haftklebemittel (2) und eine absorbierende Insel (3), die auf der gleichen Seite wie die Klebemittellage an der Trägerschicht angehaftet ist, wobei die Insel einen kleineren Bereich aufweist als die Trägerschicht, so daß ein mit Klebemittel beschichteter Rand (4, 5) der Trägerschicht um die Insel herum verläuft, wobei die Insel von dem Zentrum der Trägerschicht versetzt ist, um einen breiten Bereich (5) des mit Klebemittel beschichteten Randes bereitzustellen, **dadurch gekennzeichnet, daß** in dem breiten Bereich (5) des mit Klebemittel beschichteten Randes ein potenziell zu entfernendes Gebiet (10) kenntlich gemacht ist und das Verfahren weiterhin das Entfernen des Bereichs (10) umfaßt, um die Anpassung des Verbandes an einen Bereich eines Patienten zu verbessern.

15. Verfahren nach Anspruch 14, **dadurch gekennzeichnet, daß**
es sich bei dem Verband um einen im Wesentlichen rechteckigen Sakralverband handelt, die Insel (3) an einer kurzen Achse des Rechtecks entlang versetzt ist und es sich bei dem Bereich (10) um eine Kerbe in dem breiten, mit Klebemittel beschichteten Rand in dem Bereich der kurzen Achse handelt.

## Revendications

1. Pansement adhésif comprenant : une feuille de support (1) ; une couche d'un adhésif sensible à la pression (2) revêtue sur la feuille de support ; et un îlot absorbant (3) collé à la feuille de support sur le même côté que la couche adhésive, dans lequel l'îlot a une superficie plus petite que la feuille de support (1) de telle sorte qu'un bordure revêtue d'adhésif (4, 5) de la feuille de support s'étend autour de l'îlot, dans lequel l'îlot (3) est décalé du centre de la feuille de support, **caractérisé en ce qu'**une région potentielle qui doit être enlevée (10) est indiquée dans une partie large (5) de la bordure revêtue d'adhésif.

2. Pansement adhésif selon la revendication 1, dans lequel l'îlot (3) est décalé de 1 à 8 cm.

3. Pansement adhésif selon la revendication 1 ou 2, dans lequel la largeur moyenne de la bordure revêtue d'adhésif (4, 5) varie de 1 à 6 cm.

4. Pansement adhésif selon n'importe quelle revendication précédente, dans lequel la feuille de support (1) est substantiellement rectangulaire.

5. Pansement adhésif selon la revendication 4, dans lequel la feuille de support (1) a des dimensions allant de 5 à 25 cm par 5 à 25 cm.

6. Pansement adhésif selon la revendication 5, dans lequel la feuille de support (1) a fes dimensions allant de 10 à 20 cm par 10 à 20 cm.

7. Pansement adhésif selon n'importe quelle revendication précédente, dans lequel le pansement est un pansement pour une blessure au sacrum.

8. Pansement adhésif selon n'importe quelle revendication précédente, dans lequel au moins un bord de la feuille de support (1) est non-linéaire.

9. Pansement adhésif selon n'importe quelle revendication précédente, dans lequel la feuille de support (1) est formée avec au moins une ligne de symétrie et l'îlot (3) est décalé le long d'une ligne de symétrie de la feuille de support.

10. Pansement adhésif selon n'importe quelle revendication précédente, dans lequel une largeur de bordure adhésive minimale est indiquée (9) dans la partie large (5) de la bordure revêtue d'adhésif (4, 5).

11. Pansement adhésif selon n'importe quelle revendication précédente, comprenant en outre au moins une feuille de couverture de décollage recouvrant l'îlot (3) et la bordure revêtue d'adhésif (4, 5).

12. Pansement adhésif selon la revendication 11, comprenant deux feuilles de couverture de bord (6, 8) recouvrant les bords opposés du pansement et une feuille de couverture centrale (7) s'étendant entre les feuilles de couverture de bord.

13. Pansement adhésif selon n'importe quelle revendication précédente, dans lequel le pansement est stérile et conditionné.

14. Procédé de production d'un pansement pour blessure comprenant les étapes consistant à : fournir un pansement adhésif comprenant : une feuille de support (1) ; une couche d'un adhésif sensible à la pression (2) revêtue sur la feuille de support ; et un îlot absorbant (3) collé à la feuille de support sur le même côté que la couche adhésive, dans lequel l'îlot a une superficie plus petite que la feuille de support (1) de telle sorte qu'une bordure revêtue d'adhésif (4, 5) de la feuille de support s'étend autour de l'îlot, dans lequel l'îlot (3) est décalé du centre de la feuille de support, pour fournir une vaste région (5) de la bordure revêtue d'adhésif ; **caractérisé en ce qu'**une région potentielle qui doit être enlevée (10) est indiquée dans une partie large (5) de la bordure revêtue d'adhésif et ledit procédé comprend en outre le fait d'enlever ladite région (10) pour améliorer la conformité du pansement à une région d'un patient.

15. Procédé selon la revendication 14, dans lequel le pansement est un pansement sacral sensiblement rectangulaire, l'îlot (3) est déplacé le long d'un petit axe du rectangle et ladite région (10) est une encoche dans la large bordure revêtue d'adhésif dans la région du petit axe.
